(19) Europäisches Patentamt
European Patent Office
Office européen des brevets

(11) Veröffentlichungsnummer: **0 012 338 A1**

# (12) EUROPÄISCHE PATENTANMELDUNG

(21) Anmeldenummer: **79104922.4**

(22) Anmeldetag: **05.12.79**

(51) Int. Cl.³: **C 07 D 237/04**, A 61 K 31/50

(30) Priorität: **15.12.78 DE 2854191**

(43) Veröffentlichungstag der Anmeldung: **25.06.80 Patentblatt 80/13**

(84) Benannte Vertragsstaaten: **AT BE CH DE FR GB IT LU NL**

(71) Anmelder: **BASF Aktiengesellschaft, Carl-Bosch-Strasse 38, D-6700 Ludwigshafen (DE)**

(72) Erfinder: **Thyes, Marco, Dr., Dipl.-Chem., Mundenheimer Strasse 148, D-6700 Ludwigshafen (DE)**
Erfinder: **Gries, Josef, Dr., Roemerweg 43, D-6700 Wachenheim (DE)**
Erfinder: **Lehmann, Hans Dieter, Dr., Im Hefen 15, D-6945 Hirschberg-Leutershausen (DE)**
Erfinder: **Lenke, Dieter, Dr., Kekuleplatz 1, D-6700 Ludwigshafen (DE)**
Erfinder: **Kunze, Johannes, Dr., Pfarrweg 5, D-8343 Triftern (DE)**

(54) **Dihydropyridazinone, Verfahren zu ihrer Herstellung und diese Verbindungen enthaltende therapeutische Mittel.**

(57) Die Erfindung betrifft 6-(p-Acylaminophenyl)-4,5-dihydro-3(2H)-pyridazinone der Formel

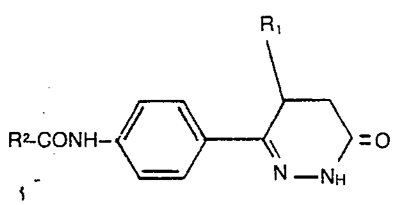

in der für R¹ Wasserstoff oder ein Alkylrest mit 1 bis 3 C-Atomen steht und R² einen durch 2 bis 6 Halogenatome substituierten Alkylrest mit 1 bis 6 C-Atomen bedeutet, ihre Herstellung und therapeutische Mittel, die diese als Wirkstoff enthalten. Die erfindungsgemässen Verbindungen können als Antihyperteniva und zur Thiophylaxe und Therapie thrombo-embolischer Erkrankungen verwendet werden.

EP 0 012 338 A1

# Dihydropyridazinone, Verfahren zu ihrer Herstellung und diese Verbindungen enthaltende therapeutische Mittel

Die Erfindung betrifft neue 6-(p-Acylaminophenyl)-4,5-dihydro-3(2H)-pyridazinone, Verfahren zu ihrer Herstellung, diese Verbindungen enthaltende pharmazeutische Zubereitungen und ihre Verwendung als Arzneimittel bei thrombo-embolischen Erkrankungen und als Antihypertensiva.

In der deutschen Patentanmeldung P 27 27 481.3 vom 18.06.77 werden 6-(p-Acylaminophenyl)-4,5-dihydro-3-(2H)-pyridazinone, die im Pyridazinonring in 5-Stellung gegebenenfalls einen Alkylrest mit 1 bis 3 C-Atomen aufweisen und im Acylrest durch ein Halogenatom substituiert sind, für die Behandlung von thrombo-embolischen Erkrankungen und zu hohen Blutdrucks vorgeschlagen.

Es wurde nun gefunden, daß 6-(p-Acylaminophenyl)-4,5-dihydro-3(2H)-pyridazinone der Formel I,

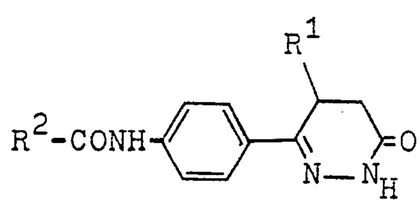

I

in der für $R^1$ Wasserstoff oder ein Alkylrest mit 1 bis 3 C-Atomen steht und $R^2$ einen durch 2 bis 6 Halogenatome substituierten Alkylrest mit 1 bis 6 C-Atomen bedeuten, wertvolle pharmakologische Eigenschaften aufweisen.

Als Alkylreste für $R^1$ seien insbesondere Methyl, Äthyl und Propyl genannt.

D/ro

0012338

Durch Halogen, wie Chlor-, Brom-, Fluor- und Jodatome, mehrfach substituierte Alkylreste für $R^2$ sind beispielsweise Dichlormethyl, Dibrommethyl, Difluormethyl, Dijodmethyl, Bromchlormethyl, Chlorfluormethyl, Chlorjodmethyl, 1,1-Dichloräthyl, 1,2-Dichloräthyl, 2,2-Dichloräthyl, 1,1-Dichlorpropyl, 1,2-Dichlorpropyl, 1,2-Dibrompropyl, 1,3-Dichlorpropyl, 1,3-Dibrompropyl, 1-Brom-3-chlorpropyl, 2,3-Dibrompropyl, 1,2-Dichlorisopropyl, 1,3-Dibrombutyl, 1,4-Dichlorbutyl, 1,4-Dibrombutyl, 1,1-Bis-chlormethyl-äthyl, 1,6-Dichlorhexyl, Trichlormethyl, Trifluormethyl, Chlordibrommethyl, Chlordifluormethyl, 1,1,2-Trichloräthyl, 2,2,2-Trifluoräthyl, 1,3,3-Trichlorpropyl, 3,3,3-Trifluorpropyl, 1,1,2,2-Tetrafluoräthyl, 2-Chlor-1,1,2-trifluoräthyl, 2,3,3,3-Tetrachlorpropyl, Pentafluoräthyl.

Die bevorzugten Bedeutungen für $R^1$ sind Wasserstoff oder Methyl. Für $R^2$ sind durch 2 oder 3 Halogenatome substituierte Methylreste und durch 2 bis 4 Halogenatome, jeweils insbesondere Chlor- oder Fluoratome, substituierte Alkylreste mit 2 bis 4 C-Atomen bevorzugt.

Die Dihydropyridazinone der Formel I können hergestellt werden, indem man eine Verbindung der Formel II,

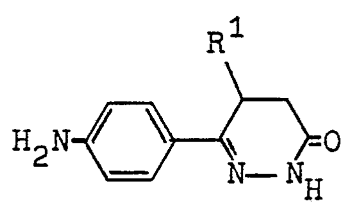

II

in der $R^1$ die für Formel I angegebenen Bedeutungen hat, mit einem Acylierungsmittel der Formel III,

$$R^2COY \qquad III$$

in der $R^2$ die für Formel I angegebenen Bedeutungen hat und für Y OH, Chlor, Brom, ein niederer Alkoxyrest oder $OCOR^2$ steht, in an sich bekannter Weise umsetzt.

Gemäß den für Y angegebenen Bedeutungen sind zweckmäßige Acylierungsmittel die entsprechenden Carbonsäuren, Carbonsäurehalogenide, insbesondere Chloride und Bromide, Carbonsäureester, insbesondere Methyl- und Äthylester, und die entsprechenden Carbonsäureanhydride.

Die Acylierung wird unter an sich üblichen Bedingungen durchgeführt. In der Regel unter Verwendung von wenigstens einer äquimolaren Menge des Acylierungsmittels, zweckmäßig in Gegenwart eines Lösungsmittels und gegebenenfalls in Gegenwart einer Hilfsbase bei Temperaturen zwischen 0 und 160°C, gegebenenfalls bei den Siedetemperaturen des Reaktionsgemisches und gegebenenfalls unter Anwendung von Druck.

Als Lösungsmittel kommen unter den Reaktionsbedingungen inerte Lösungsmittel, wie aromatische Kohlenwasserstoffe, beispielsweise Benzol oder Toluol, cyclische aliphatische Äther, wie Dioxan, oder Dialkylformamide, wie Dimethylformamid, in Betracht. Hilfsbasen als säurebindende Mittel sind zweckmäßigerweise anorganische Basen, wie Natrium- oder Kaliumcarbonat, Natrium- oder Kalium-hydrogencarbonat, oder tertiäre organische Amine, wie Triäthylamin.

Die Herstellung der erfindungsgemäßen 6-(p-Acylamino-phenyl)-4,5-dihydro-3(2H)-pyridazinone der Formel I wird durch das folgende Formelschema veranschaulicht:

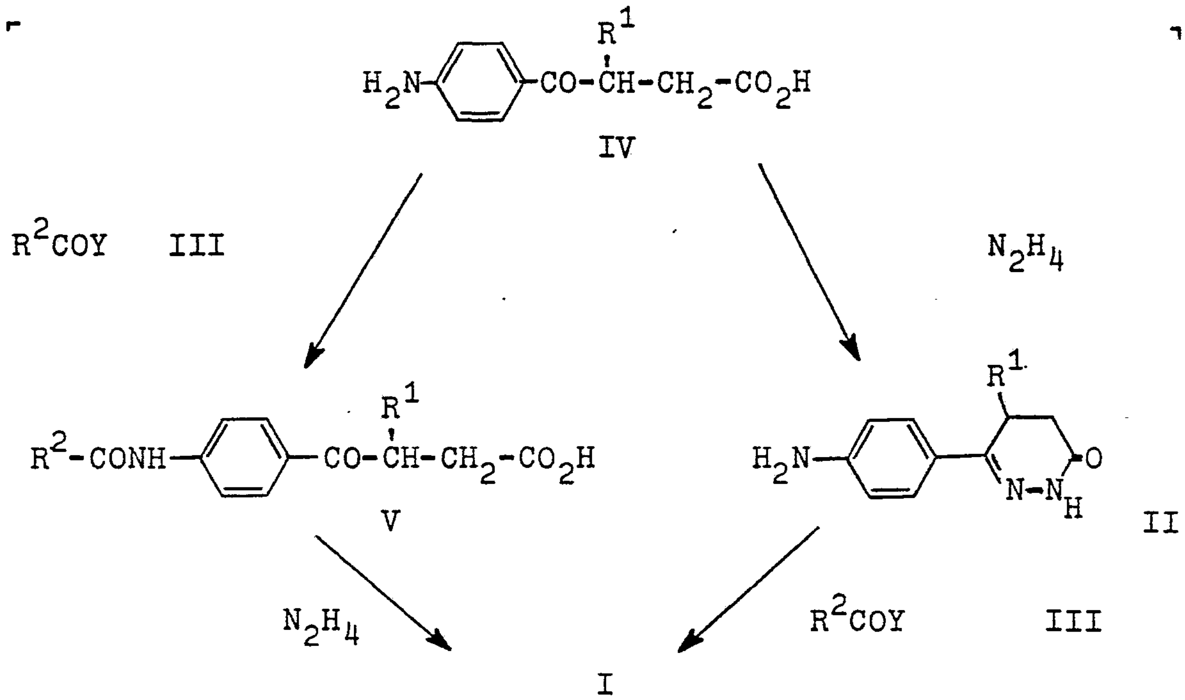


Demnach werden gemäß einer weiteren Ausführungsform die Dihydropyridazinone der Formel I hergestellt, indem man eine Aminosäure der Formel IV, in der $R^1$ die für Formel I angegebenen Bedeutungen hat, mit einem Acylierungsmittel der Formel III,

$R^2COY$ III

in der $R^2$ und Y die oben angegebenen Bedeutungen haben, unter den oben angegebenen Bedingungen acyliert und die erhaltene Acylaminoverbindung der Formel V

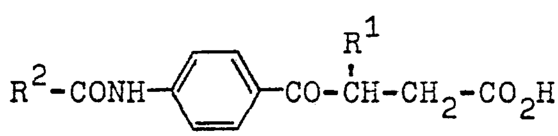
V

mit Hydrazin cyclisiert.

Die Ringschlußreaktion mit Hydrazin oder Hydrazinhydrat wird vorteilhaft mit einer äquimolaren Menge Hydrazin in einem Lösungsmittel, insbesondere einem niederen Alkohol, wie Methanol, Äthanol oder Propanol, einem cyclischen aliphatischen Äther, wie Dioxan, oder einem Dialkylformamid, wie Dimethylformamid, bei Temperaturen von 60 bis 150°C, vorzugsweise 80 bis 120°C, durchgeführt.

Die als Ausgangssubstanzen verwendeten Verbindungen der Formel II und auch der Formel IV sind bekannt oder können beispielsweise unter den in den DE-OS 16 70 158 und 21 50 436 oder den US-PS 3 824 271 und 3 888 901 beschriebenen Bedingungen hergestellt werden.

Erfindungsgemäße Verbindungen, die nach den genannten Verfahren erhalten werden, sind beispielsweise:

6-(p-Dichloracetylaminophenyl)-4,5-dihydro-3(2H)-pyridazinon;
6-(p-Dichloracetylaminophenyl)-4,5-dihydro-5-methyl-3(2H)-pyridazinon;
6-(p-Dibromacetylaminophenyl)-4,5-dihydro-5-methyl-3(2H)-pyridazinon;
6-(p-Difluoracetylaminophenyl)-4,5-dihydro-3(2H)-pyridazinon;
6-(p-Difluoracetylaminophenyl)-4,5-dihydro-5-methyl-3(2H)-pyridazinon;
4,5-Dihydro-6-(p-dijodacetylaminophenyl)-5-methyl-3(2H)-pyridazinon;
6-(p-Bromchloracetylaminophenyl)-4,5-dihydro-5-methyl-3(2H)-pyridazinon;
6-[p-(2,2-Dichlorpropionylamino)phenyl]-4,5-dihydro-5-methyl-3(2H)-pyridazinon;
6-[p-(2,3-Dichlorpropionylamino)phenyl]-4,5-dihydro-3(2H)-pyridazinon;
6-[p-(2,3-Dichlorpropionylamino)phenyl]-4,5-dihydro-5-methyl-3(2H)-pyridazinon;

6-[p-(3,3-Dichlorpropionylamino)phenyl]-4,5-dihydro-5-methyl-3(2H)-pyridazinon;
6-[p-(2,2-Dichlorbutyrylamino)phenyl]-4,5-dihydro-5-methyl-3(2H)-pyridazinon;
6-[p-(2,3-Dichlorbutyrylamino)phenyl]-4,5-dihydro-5-methyl-3(2H)-pyridazinon;
6-[p-(2,4-Dichlorbutyrylamino)phenyl]-4,5-dihydro-5-methyl-3(2H)-pyridazinon;
6-[p-(3,4-Dibrombutyrylamino)phenyl]-4,5-dihydro-5-methyl-3(2H)-pyridazinon;
6-[p-(2,3-Dichlorisobutyrylamino)phenyl]-4,5-dihydro-5-methyl-3(2H)-pyridazinon;
6-[p-(2,4-Dibromvalerylamino)phenyl]-4,5-dihydro-5-methyl-3(2H)-pyridazinon;
6-[p-(2,5-Dichlorvalerylamino)phenyl]-4,5-dihydro-5-methyl-3(2H)-pyridazinon;
4,5-Dihydro-6-(p-trichloracetylaminophenyl)-3(2H)-pyridazinon;
4,5-Dihydro-6-(p-trifluoracetylaminophenyl)-3(2H)-pyridazinon;
4,5-Dihydro-5-methyl-6-(p-trifluoracetylaminophenyl)-3(2H)-pyridazinon;
6-(p-Chlordifluoracetylaminophenyl)-4,5-dihydro-3(2H)-pyridazinon;
6-(p-Chlordifluoracetylaminophenyl)-4,5-dihydro-5-methyl-3(2H)-pyridazinon;
4,5-Dihydro-5-methyl-6-[p-(2,2,3-trichlorpropionylamino)-phenyl]-3(2H)-pyridazinon;
4,5-Dihydro-5-methyl-6-[p-(3,3,3-trifluorpropionylamino)-phenyl]-3(2H)-pyridazinon;
4,5-Dihydro-5-methyl-6-[p-(2,4,4-trichlorbutyrylamino)-phenyl]-3(2H)-pyridazinon;
4,5-Dihydro-5-methyl-6-[p-(2,2,3,3-tetrafluorpropionylamino)phenyl]-3(2H)-pyridazinon;
4,5-Dihydro-5-methyl-6-[p-(3,4,4,4-tetrachlorbutyrylamino)-phenyl]-3(2H)-pyridazinon.

Es wird darauf hingewiesen, daß die Verbindungen der Formel I, in denen $R^1$ ungleich Wasserstoff ist, ein asymmetrisches Kohlenstoffatom in 5-Stellung aufweisen und als Racemate erhalten werden. Die vorliegende Erfindung soll die Enantiomeren mit einschließen. Ist eine Trennung erwünscht, so wird diese vorteilhaft auf der Stufe einer Verbindung der Formel II nach bekannten Methoden, z.B. Bildung diastereomerer Salze mit optisch aktiven Hilfssäuren, wie Dibenzoylweinsäure oder Campher-10-sulfonsäure, durchgeführt.

Die erfindungsgemäßen Dihydropyridazinone der Formel I zeichnen sich durch thrombozytenaggregationshemmende und blutdrucksenkende Eigenschaften aus. Sie sind als Antihypertensiva und zur Prophylaxe und Therapie thrombo-embolischer Erkrankungen geeignet.

Die vorteilhafte thrombozytenaggregationshemmende Wirkung kann im Vergleich zu Acetylsalicylsäure z.B. an der durch Collagen induzierten Aggregation von Thrombozyten des Menschen festgestellt werden. Zum Nachweis der blutdrucksenkenden Wirkung werden z.B. Ratten in Urethan-Narkose verwendet. Als Referenzsubstanz dient hier Dihydralazin.

Gegenstand der vorliegenden Erfindung sind demnach auch therapeutische Mittel oder Zubereitungen, die neben pharmazeutisch üblichen Träger- und Verdünnungsmitteln eine Verbindung der Formel I als Wirkstoff enthalten, sowie die Verwendung dieser Verbindungen zu therapeutischen Zwecken.

Die therapeutischen Mittel oder Zubereitungen werden mit den üblichen Trägerstoffen oder Verdünnungsmitteln und den üblicherweise verwendeten pharmazeutisch technischen Hilfsstoffen entsprechend der gewünschten Applikationsart mit einer geeigneten Dosierung in bekannter Weise herge-

stellt. Dabei kommen beim Menschen Dosen von 1 bis 100 mg, bevorzugt 5 bis 50 mg, in Betracht, wobei die orale Applikation bevorzugt ist.

Darreichungsformen, die zur oralen Applikation geeignet sind, sind beispielsweise Tabletten, Filmtabletten, Dragees, Kapseln, Pillen, Pulver, Lösungen, Suspensionen oder Depotformen.

Zur praktischen Anwendung werden die erfindungsgemäß zu verwendenden Verbindungen mit den in der galenischen Pharmazie üblichen flüssigen oder festen Trägerstoffen verarbeitet. Die entsprechenden Tabletten können beispielsweise durch Mischen des Wirkstoffs mit bekannten Hilfsstoffen, beispielsweise inerten Verdünnungsmitteln, wie Dextrose, Zucker, Sorbit, Polyvinylpyrrolidon, Mannit, Calciumcarbonat, Calciumphosphat oder Milchzucker, Sprengmitteln, wie Mais, Stärke, Alginsäure oder Polyvinylpyrrolidon, Bindemitteln, wie Stärke oder Gelatine, Gleitmitteln, wie Magnesiumstearat oder Talkum, und/oder Mitteln zur Erzielung des Depoteffektes, wie Carboxypolymethylen, Carboxymethylcellulose, Celluloseacetatphthalat oder Polyvinylacetat, erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Polyvinylpyrrolidon oder Schellack, Gummi arabicum, Talk, Titandioxid oder Zucker, hergestellt werden. Dabei kann auch die Drageehülle aus mehreren Schichten bestehen, wobei die oben bei den Tabletten erwähnten Hilfsstoffe verwendet werden können.

Die Herstellung der neuen 6-(p-Acylaminophenyl)-4,5-dihydro-3(2H)-pyridazinone wird durch die folgenden Beispiele näher erläutert.

Beispiel 1

6,0 g (31,7 mmol) 6-(p-Aminophenyl)-4,5-dihydro-3(2H)-pyridazinon werden mit 5,1 g (34,6 mmol) Dichloracetylchlorid in 100 ml absolutem Toluol 6 Stunden bei 80°C gehalten. Man saugt bei 10°C ab, wäscht zuerst mit Toluol, dann mit Wasser und kristallisiert zweimal aus Dimethylformamid/Wasser um. Nach dem Trocknen unter vermindertem Druck bei 100°C erhält man 7,7 g (81 % d.Th.) 6-(p-Dichloracetylaminophenyl)-4,5-dihydro-3(2H)-pyridazinon, beige Kristalle. Schmelzpunkt 250 bis 251°C (Zers.).

Analyse für $C_{12}H_{11}Cl_2N_3O_2$,

| | | | | | |
|---|---|---|---|---|---|
| ber.: | C | 48,0 | H 3,7 | Cl 23,6 | N 14,0 % |
| gef.: | C | 47,5 | H 4,0 | Cl 23,3 | N 13,7 %. |

Beispiel 2

5,0 g (24,6 mmol) 6-(p-Aminophenyl)-4,5-dihydro-5-methyl-3(2H)-pyridazinon werden mit 15 ml Trifluoressigsäureanhydrid 15 Minuten bei 80°C gehalten. Man versetzt das Reaktionsgemisch mit 300 ml Eiswasser und saugt ab. Nach dem Umkristallisieren aus Methanol isoliert man 1,9 g (26 % d.Th.) 4,5-Dihydro-5-methyl-6-(p-trifluoracetylaminophenyl)-3(2H)-pyridazinon, farblose Kristalle, Schmelzpunkt 255 bis 257°C.

Analyse für $C_{13}H_{12}F_3N_3O_2$:

| | | | | |
|---|---|---|---|---|
| ber.: | C 52,2 | H 4,0 | F 19,0 | N 14,0 % |
| gef.: | C 52,7 | H 4,2 | F 19,0 | N 14,3 % |

Die Herstellung der in der folgenden Tabelle angegebenen Dihydropyridazinone erfolgt nach der im Beispiel 1 beschriebenen Methode.

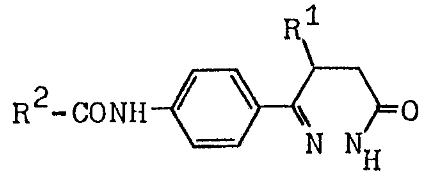

| Beispiel | $R^1$ | $R^2$ | Schmp. [°C] | | Analyse (%) C | H | Cl | N |
|---|---|---|---|---|---|---|---|---|
| 3 | $-CH_3$ | 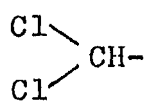 | 245-247 (Dimethyl-formamid/Wasser) | ber.: | 49.7 | 4,2 | 22,6 | 13,4 |
| | | | | gef.: | 49,6 | 4,2 | 22,6 | 13,4 |
| 4 | $-CH_3$ | 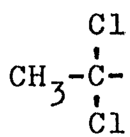 | 218-219 (Propanol) | ber.: | 51,2 | 4,6 | 21,6 | 12,8 |
| | | | | gef.: | 51,5 | 4,6 | 21,1 | 12,9 |
| 5 | -H | 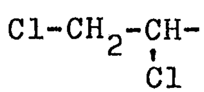 | 199-202 (Zers.) (Dimethylform-amid/Wasser) | ber.: | 49,7 | 4,2 | 22,6 | 13,4 |
| | | | | gef.: | 49,5 | 4,4 | 21,9 | 12,9 |
| 6 | $-CH_3$ | 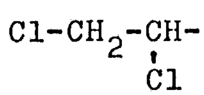 | 213-214 (Zers.) (Dimethylform-amid/Wasser) | ber.: | 51,2 | 4,6 | 21,6 | 12,8 |
| | | | | gef.: | 51,3 | 4,6 | 21,6 | 12,7 |
| 7 | -H | 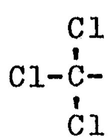 | 232-234 (Zers.) (Dimethylform-amid/Wasser) | ber.: | 43,1 | 3,0 | 31,8 | 12,6 |
| | | | | gef.: | 43,4 | 3,2 | 31,6 | 12,7 |

Beispiel 8

5,0 g (24,6 mmol) 6-(p-Aminophenyl)-4,5-dihydro-5-methyl-3(2H)-pyridazinon werden mit 2,95 g (25,8 mmol) Difluoracetylchlorid und 100 ml absolutem Tetrahydrofuran zuerst 3 Stunden bei 0 bis 5°C und dann noch 20 Stunden bei Raumtemperatur gehalten. Man saugt bei 10°C ab, wäscht mit Wasser und kristallisiert aus Methanol um. Man erhält 4,2 g (61 % d.Th.) 6-(p-Difluormethylaminophenyl)-4,5-dihydro-5-methyl-3(2H)-pyridazinon, farblose Kristalle, Schmelzpunkt 236 bis 237°C.

Analyse für $C_{13}H_{13}F_2N_3O_2$:

| | | | | | | | | |
|---|---|---|---|---|---|---|---|---|
| ber.: | C | 55,5 | H | 4,7 | F | 13,5 | N | 14,9 % |
| gef.: | C | 55,5 | H | 4,7 | F | 13,5 | N | 14,9 %. |

Beispiel 9

6,0 g (31,7 mmol) 6-(p-Aminophenyl)-4,5-dihydro-3(2H)-pyridazinon werden mit 40 ml Trifluoressigsäureanhydrid 45 Minuten bei Rückflußtemperatur gehalten. Man saugt bei 10°C ab, wäscht zuerst mit Toluol, dann mit Wasser und kristallisiert aus Dimethylformamid/Wasser um. Man isoliert 5,8 g (64 % d.Th.) 4,5-Dihydro-6-(p-trifluoracetylaminophenyl)-3(2H)-pyridazinon, farblose Kristalle, Schmelzpunkt 242 bis 244°C.

Analyse für $C_{12}H_{10}F_3N_3O_2$:

| | | | | | | | | |
|---|---|---|---|---|---|---|---|---|
| ber.: | C | 50,5 | H | 3,5 | F | 20,0 | N | 14,7 % |
| gef.: | C | 50,4 | H | 3,7 | F | 19,8 | N | 14,7. |

Formulierungsbeispiele, die in üblicher Weise hergestellt werden:

1. <u>Tabletten:</u>

| | |
|---|---|
| Wirkstoff | 10 mg |
| Polyvinylpyrrolidon (mittl. M.G. 25 000) | 170 mg |
| Polyäthylenglykol (mittl. M.G. 4 000) | 14 mg |
| Hydroxypropylmethylcellulose | 40 mg |
| Talkum | 4 mg |
| Magnesiumstearat | 2 mg |
| | 240 mg |

Der Wirkstoff wird mit Polyvinylpyrrolidon in 10 %iger wäßriger Lösung befeuchtet, durch ein Sieb mit der lichten Maschenweite 1,0 mm getrieben und bei 50°C getrocknet. Dieses Granulat wird mit Polyäthylenglykol (mittl. M.G. 4 000), Hydroxypropylmethylcellulose, Talkum und Magnesiumstearat vermischt und zu Tabletten â 240 mg verpreßt.

2. <u>Beispiel für Dragees:</u>

| | |
|---|---|
| Wirkstoff | 10 mg |
| Lactose | 90 mg |
| Maisstärke | 60 mg |
| Polyvinylpyrrolidon | 6 mg |
| Magnesiumstearat | 1 mg |
| | 167 mg |

Die Mischung der Wirkstoffsubstanz mit Lactose und Maisstärke wird mit einer 8 %igen wäßrigen Lösung des Polyvinylpyrrolidons durch Sieb 1,5 mm granuliert, bei 50°C getrocknet und nochmals durch Sieb 1,0 mm getrieben. Das so erhaltene Granulat wird mit Magnesiumstearat gemischt und zu Drageekernen verpreßt. Die erhaltenen Drageekerne werden in üblicher Weise mit einer Hülle überzogen, die im wesentlichen aus Zucker und Talkum besteht.

Patentansprüche

1. 6-(p-Acylaminophenyl)-4,5-dihydro-3(2H)-pyridazinone der Formel I,

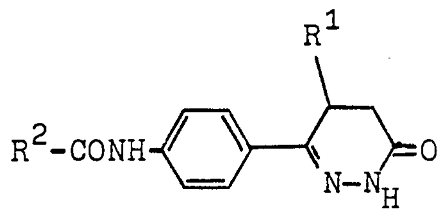

in der für $R^1$ Wasserstoff oder ein Alkylrest mit 1 bis 3 C-Atomen steht und $R^2$ einen durch 2 bis 6 Halogenatome substituierten Alkylrest mit 1 bis 6 C-Atomen bedeutet.

2. Verbindungen der Formel I, in denen $R^1$ Wasserstoff oder Methyl und $R^2$ einen durch 2 oder 3 Halogenatome substituierten Methylrest oder einen durch 2 bis 4 Halogenatome substituierten Alkylrest mit 2 bis 4 C-Atomen bedeuten.

3. Verfahren zur Herstellung von Verbindungen der Formel I, dadurch gekennzeichnet, daß man eine Verbindung der Formel II,

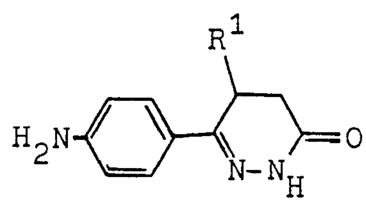

II

in der $R^1$ die für Formel I angegebene Bedeutungen hat, mit einem Acylierungsmittel der Formel III,

$R^2COY$ III

in der $R^2$ die für Formel I angegebenen Bedeutungen hat und für Y OH, Chlor, Brom, ein niederer Alkoxyrest oder der Rest $OCOR^2$ steht, gegebenenfalls in einem Lösungsmittel und gegebenenfalls in Gegenwart einer Base umsetzt oder daß man eine Verbindung der Formel V

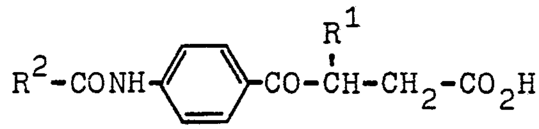 V

mit Hydrazin cyclisiert.

4. Therapeutisches Mittel, gekennzeichnet durch einen Gehalt an einer Verbindung der Formel I nach Anspruch 1 als Wirkstoff neben üblichen Trägerstoffen und Verdünnungsmitteln.

5. Verwendung einer Verbindung der Formel I bei der Bekämpfung thrombo-embolischer Erkrankungen.

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

Nummer der Anmeldung

0012338

EP 79 10 4922

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | FR - A - 2 137 745 (BASF)<br>* Vollständig *<br>-- | 1-4 |
| PD | EP - A - 0 000 113 (BASF)<br>* Seiten 22,23, Ansprüche *<br>---- | 1-4 |

KLASSIFIKATION DER ANMELDUNG (Int. Cl.³)

C 07 D 237/04
A 61 K 31/50

RECHERCHIERTE SACHGEBIETE (Int. Cl.³)

C 07 D 237/04

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-4

Unvollständig recherchierte Patentansprüche:

Nicht recherchierte Patentansprüche: 5

Grund für die Beschränkung der Recherche: Verfahren zur chirurgischen oder therapeutischen Behandlung des menschlichen oder tierischen Körpers. (Siehe Art. 52(4) des Europäischen Patentübereinkommens).

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

| Recherchenort | Abschlußdatum der Recherche | Prüfer |
|---|---|---|
| Den Haag | 03-03-1980 | ALLARD |